# EUROPEAN PATENT SPECIFICATION

(11) **EP 4 247 400 B1**
(45) Date of publication and mention of the grant of the patent: **30.09.2026**
(21) Application number: 21893156.6
(22) Date of filing: 23.11.2021
(51) Int. Cl.: A61K 36/899, A61P 31/04, A61P 31/12, A61P 1/14, A61P 33/02, A23K 10/33, A23K 20/00, A23K 50/80

(54) **USE OF SUGAR CANE EXTRACTS IN REDUCING METHANE PRODUCTION BY A RUMINANT**
VERWENDUNG VON ZUCKERROHR-EXTRAKTEN ZUR VERRINGERUNG DER METHANPRODUKTION BEI EINEM WIEDERKÄUER
UTILISATION D'EXTRAITS DE CANNE À SUCRE POUR RÉDUIRE LA PRODUCTION DE MÉTHANE PAR UN RUMINANT

(30) Priority: 23.11.2020 AU 2020904323
(43) Date of publication of application: 27.09.2023
(73) Proprietor: Poly Gain Pte Ltd, Singapore 408555 (SG)
(72) Inventor: MITCHELL, Shane, Keysborough, Victoria 3173 (AU); KITCHEN, Barry, Keysborough, Victoria 3173 (AU); MACNAB, Gregor, Keysborough, Victoria 3173 (AU); FLAVEL, Matthew, Keysborough, Victoria 3173 (AU)
(74) Representative: Mewburn Ellis LLP
(86) International application number: PCT/AU2021/051395
(87) International publication number: WO 2022/104438

(56) References cited:
- EP-A1- 1 120 118
- WO-A1-2012/106761
- WO-A1-2014/032100
- WO-A1-2019/028506
- WO-A1-2019/213703
- WO-A2-2023/277820
- JP-A- 2003 063 975
- US-A1- 2009 285 931
- US-B2- 9 572 852
- KENJI KOGE ET AL: "Antioxidants and Other Functional Extracts from Sugarcane", ASIAN FUNCTIONAL FOODS, 3 March 2005 (2005-03-03), XP055651257, ISBN: 978-1-4200-2811-9, DOI: 10.1201/9781420028119.ch15
- BARRERA CRISTINA ET AL: "Phenolic Profile of Cane Sugar Derivatives Exhibiting Antioxidant and Antibacterial Properties", SUGAR TECH, SPRINGER INDIA, NEW DELHI, vol. 22, no. 5, 31 March 2020 (2020-03-31), pages 798 - 811, XP037222300, ISSN: 0972-1525, [retrieved on 20200331], DOI: 10.1007/S12355-020-00817-Y
- AHTESH FATAH B ET AL: "Polyphenol Rich Sugar Cane Extract Inhibits Bacterial Growth", PRILOZI, vol. 41, no. 3, 1 November 2020 (2020-11-01), pages 49 - 57, XP055940296, ISSN: 1857-9345, DOI: 10.2478/prilozi-2020-0045
- WIJESIRIWARDANA UDANI A. ET AL: "Evaluation of Sugarcane-Derived Polyphenols on the Pre-Weaning and Post-Weaning Growth of Gilt Progeny", ANIMALS, vol. 10, no. 6, 5 June 2020 (2020-06-05), pages 984, XP093203066, ISSN: 2076-2615, DOI: 10.3390/ani10060984
- GOMES FERNANDA, MARTINS NATÁLIA, BARROS LILLIAN, RODRIGUES MARIA ELISA, OLIVEIRA M. BEATRIZ P.P., HENRIQUES MARIANA, FERREIRA ISAB: "Plant phenolic extracts as an effective strategy to control Staphylococcus aureus , the dairy industry pathogen", INDUSTRIAL CROPS AND PRODUCTS, ELSEVIER, NL, vol. 112, 1 February 2018 (2018-02-01), NL , pages 515 - 520, XP055940290, ISSN: 0926-6690, DOI: 10.1016/j.indcrop.2017.12.027
- WIJAYANTI CHANDRA, HAPSARI NADIA RIZKY PUTRI, MARIANA RINA RIFQIE, SUHARTI SUHARTI, SUBANDI SUBANDI: "Total phenolic and total flavonoid content of sugar cane juice and their potency as alpha-amylase inhibitor and anti COVID-19", AIP CONFERENCE PROCEEDINGS, AMERICAN INSTITUTE OF PHYSICS, NEW YORK, US, vol. 2353, 1 January 2021 (2021-01-01), NEW YORK, US , pages 030079, XP055940291, ISSN: 0094-243X, DOI: 10.1063/5.0052656
- AHTESH FATAH B, STOJANOVSKA LILY, FEEHAN JACK, DE COURTEN MAXIMILIAN PJ, FLAVEL MATTHEW, KITCHEN BARRY, APOSTOLOPOULOS VASSO: "Polyphenol Rich Sugar Cane Extract Inhibits Bacterial Growth", PRILOZI, vol. 41, no. 3, 1 November 2020 (2020-11-01), pages 49 - 57, XP055940296, ISSN: 1857-9345, DOI: 10.2478/prilozi-2020-0045

## Description

### Technical Field

The present disclosure is in the field of reducing methane production by ruminant animals, in particular the use of extracts derived from sugar cane comprising polyphenols for the reduction of methane production by a ruminant animal.

### Background

Animal parasitism is responsible for a significant burden on animal welfare, which associated with economic costs spanning the agricultural industry. Three major groups of parasites are generally recognised including; protozoa, helminths and arthropods.

There are a diverse range of protozoan parasites, which are known to cause disease across both vertebrate and invertebrate species. These parasites are the causative agent for diseases including, but not limited to malaria; dysentery, giardia, cryptosporidium, Chaga's disease and coccidiosis.

Whilst some protozoa are specific to one host species, other protozoa are capable of infecting a range of host species. Therefore, the unchecked usage of antimicrobial drugs, particularly throughout the food supply chain is facing growing restrictions. This to ensure that these drugs are effective in emergency situations.

The requirement for non-antibiotic alternatives to help prevent disease developing is therefore an important means to improving global health and welfare outcomes, without destroying economic viability.

Microsporidian parasites are of economic significance in aqueous environments, particularly in prawn and shrimp aquaculture. *Enterocytozoon hepatopenaei* (EHP) is known to infect only the tubule epithelial cells of the hepatopancreas of prawns. Infection occurs by the extrusion or growth of the polar tubule of the microsporidia allowing it to attach to the hepatopancreas of prawns.

Some prawn species that are particularly susceptible to EHP are *Penaeus monodon, Penaeus (Litopenaeus) vannamei and Penaeus (Litopenaeus) stylirostris.*

An effective prevention or treatment means for infections, such as EHP, would be of considerable benefit in aquaculture industries.

Microbial agents are a threat to life on earth beyond the ability to cause disease in a wide variety of living organisms. This is because methanogenic Archaea are responsible for the largest contribution to agricultural greenhouse gas emissions.

Methanogens are not associated with a disease state or parasitism in animals from which they are isolated. However, the production of gases such as methane during fermentation has been identified as a factor involved in increased greenhouse gas emissions and global warming. Therefore methanogens are damaging the planet through their effect on the environment, rather than damaging individual animals through disease.

Ruminants produce the majority of agricultural methane, but are also vital for global food and textile supply. Therefore strategies are required that are able to mitigate the methane output, whilst maintaining or improving production yields.

Dietary strategies to modulate the microbiome of animals to inhibit the proliferation of methanogens, whilst promoting the communities of microbial species that support the health of the animal would meet these objectives.

Milk producing ruminants are subject to the development of mastitis. Mastitis is a bacterial infection that causes an inflammation of a cow's udder. The infection is caused by a wide spectrum of pathogens and, epidemiologically categorized into contagious and environmental mastitis. Mastitis is the most widespread and costly disease in the dairy cattle occurring throughout the world.

Electrical conductivity (EC) of milk has been introduced as an indicator trait for mastitis over the last decade. The EC is determined by the concentration of anions and cations. If the cow suffers from mastitis, the concentration of Na+ and Cl- in the milk increases, which leads to increased electrical conductivity of milk from the infected quarter. Most automatic milking systems have EC sensors incorporated for measuring EC during milking.

A description of the utility of the measurement of electrical conductivity is to be found in J Dairy Sci. 75, 606-614 (1992).

Electrical conductivity measurements in milk from healthy cows are generally between 4.0 and 5.0 milliSiemens (mS) at 25°C, while absolute electrical conductivity values for infected quarters usually range from 5.0 to 9.0 mS. In a typical EC profile for a cow infected with mastitis, the infected quarter shows a higher EC level during most of the milking, with spikes in the beginning and at the end of the milking.

A mild, uncomplicated mastitis in a single quarter is usually treated with intramammary antibiotics, such as β-lactams (amoxicillin, penicillin, and cephalosporins). Systemic antibiotics are used when more than one quarter is infected, when udder changes are marked or when the cow is obviously ill. Combination therapy, with both systemic and intramammary antibiotics, is believed to increase bacteriological cure rates and is used in severe cases of mastitis.

The main components of the economic impact of mastitis are related to the reduction in milk production, milk disposal, the cost of the medicines used in the treatment, the labour costs related to the treatment and the culling of animals. Together, these factors erode the milk income received by farmers. It is estimated that more than $150 million is lost to Australian dairy farmers each year through poor udder health. Moreover the use of antibiotics is now discouraged owing to the development of bacterial resistance and possible undesirable antibiotic residues in the meat and milk of animals for human consumption.
US 9,572,852 describes compositions from sugar cane extracts with high polyphenol contents which may be administered to livestock to improve the health of the animal. WO 2019/213703 also describes compositions for animals, including ruminants, comprising an extract derived from sugar cane which comprises high amounts of polyphenols.

### Summary

The present invention is a use of a composition in reducing methane production by a ruminant animal by changing the composition of microbial communities associated with the rumen of the ruminant animal comprising administering to the ruminant animal, an effective amount of a composition comprising from about 0.001 wt% to about 10 wt % of an extract derived from sugar cane to reduce the methane production in the ruminant animal by 3-80%, the extract comprising from about 10 catechin equivalent (CE) g/L to about 100 CE g/L of polyphenols or from about 100 CE mg/g to about 1000 CE mg/g of polyphenols.

### Brief Description of Drawings

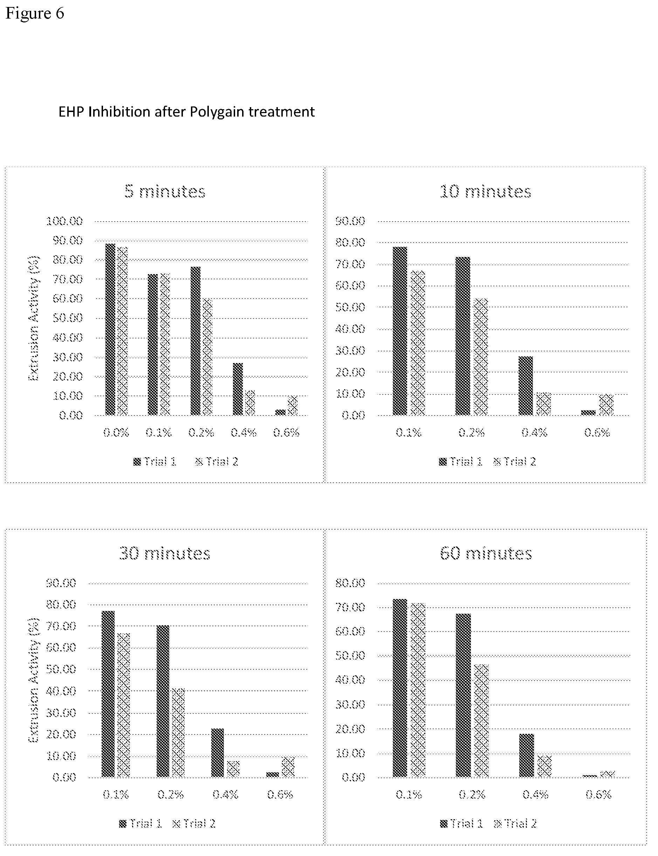

Figure 6 is a bar chart showing the results for Example 14 as described herein.
Figure 7 is a bar chart showing the results for Example 13 as described herein.
Figure 8 is a graph representing the results achieved in Example 19 for a 0.5% composition.
Figure 9 is a graph representing the results achieved in Example 19 for a 0.5% composition.
Figure 10 are bar charts showing the results for Example 20 as described herein.

### Description of Embodiments

Any embodiment herein shall be taken to apply mutatis mutandis to any other embodiment unless specifically stated otherwise.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Unless specifically defined otherwise, all technical and scientific terms used herein shall be taken to have the same meaning as commonly understood by one of ordinary skill in the art (e.g., chemistry, biochemistry, cell culture, molecular biology and pharmacy). Further, unless otherwise required by context, singular terms shall include pluralities and plural terms shall include the singular. Thus, as used in this specification and the appended claims, the singular forms "a", "an" and "the" include plural referents unless the context clearly indicates otherwise. Thus, the term "an subject" means "one or more subjects" unless the context clearly indicates otherwise.

"Administering" as used herein is to be construed broadly and includes administering an extract or a composition comprising the extract as described herein to a subject as well as providing an extract or composition comprising the extract as described herein to a cell.

The phrase "an effective amount" as used herein, refers to an amount which is sufficient to elicit the biological or medical response of a tissue, system, animal or human that is being sought by the researcher, veterinarian, medical doctor or other clinician. Undesirable effects, e.g. side effects, are sometimes manifested along with the desired effect; hence, a practitioner balances the potential benefits against the potential risks in determining what an appropriate "effective amount" is. The exact amount required varies from subject to subject, depending on the species, age and general condition of the subject, mode of administration and the like. Thus, it may not be possible to specify an exact "effective amount". However, an appropriate "effective amount" in any individual case may be determined by one of ordinary skill in the art using routine experimentation. By "ameliorate" is included relieving of adverse symptoms, inducing a state of comfort or wellbeing or removing or reducing biochemical, physiological or clinical markers of the disease or the condition.

The terms "treating", "treat", "treatment", "improving", "improve" or "improvement", as used herein, include administering an effective amount of an extract of the present disclosure or a composition comprising the extract sufficient to reduce or delay the onset or progression of a specified condition, or to reduce or eliminate at least one symptom of the condition. As would be understood by those skilled in the art of treating a microbial infection, the term "treatment" includes that the infection is cured, however, it does not necessarily mean that the infection is completely cured.

The terms "preventing" or "prevent" as used herein, include administering an effective amount of an extract of the present disclosure or a composition comprising the extract sufficient to avoid the onset of a specified condition, or to avoid at least one symptom of the condition. As would be understood by those skilled in the art of preventing a condition, the term "preventing" includes that the condition is completely prevented, however, it does not necessarily mean that the condition is completely prevented.

"Subject" as used herein refers to an animal, such as mammal including a human who can benefit from the extracts derived from sugar cane, compositions containing the extracts and methods and uses described herein. There is no limitation on the type of animal that could benefit from the presently described extracts derived from sugar cane, compositions containing the extracts and methods and uses. A subject regardless of whether a human or non-human animal may be referred to as an individual, subject, animal, host or recipient as well as patient. of the present disclosure have applications in human medicine, human cosmetics, and veterinary medicine.

References to "compositions for use" are to be understood to encompass methods of this disclosure.

The term "about" as used herein refers to a range of +/-5% of the specified value.

The term "CE", or "catechin equivalent" as used herein is a measure of total polyphenolic content, expressed as mg catechin equivalents/g crude material or g catechin equivalents/L crude material.

The term "sugar cane derived product" as used herein refers to products of the sugar cane milling and refining processes including, but not limited to, sugar, molasses, massecuite, bagasse, first expressed juice, mill mud, clarified sugar cane juice, clarified syrup, treacle, golden syrup, field trash, cane strippings, leaves, growing tips, pulp and dunder and combinations thereof. Dunder is the residue produced when a product such as sugar or molasses is fermented to give, for example, ethanol. Sugar cane dunder is also referred to as biodunder, stillage or vinasse. As used herein , the terms "dunder", "bio-dunder", "stillage" and "vinasse" are equivalent and used interchangeably.

Throughout this specification, various aspects and components of this disclosure can be presented in a range format. The range format is included for convenience and should not be interpreted as an inflexible limitation on the scope of this disclosure. Accordingly, the description of a range should be considered to have specifically disclosed all the possible sub-ranges as well as individual numerical values within that range, unless specifically indicated. For example, description of a range such as from 1 to 5 should be considered to have specifically disclosed sub-ranges such as from 1 to 3, from 1 to 4, from 1 to 5, from 2 to 4, from 2 to 5, from 3 to 5 etc., as well as individual and partial numbers within the recited range, for example, 1, 2, 3, 4, 5, 5.5 and 6, unless where integers are required or implicit from context. This applies regardless of the breadth of the disclosed range. Where specific values are required, these will be indicated in the specification.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps. In one embodiment, the extract derived from sugar cane of the present disclosure comprises at least about 10 CE g/L of polyphenols or at least about 150 mg CE/g of polyphenols. As explained above, the term "CE", or "catechin equivalent" is a measure of total polyphenolic content, expressed as catechin equivalents mg/g extract derived from sugar cane or catechin equivalents g/L extract derived from sugar cane.

The extract derived from sugar cane of the present invention comprises from about 10 CE g/L to about 100 CE g/L of polyphenols or from about 100 CE mg/g to about 1000 CE mg/g of polyphenols.

In one embodiment, the extract derived from sugar cane of the present disclosure comprises from about 10 CE g/L to about 90 CE g/L of polyphenols or from about 100 CE mg/g to about 900 CE mg/g of polyphenols.

In one embodiment, the extract derived from sugar cane of the present disclosure comprises from about 10 CE g/L to about 80 CE g/L of polyphenols or from about 100 CE mg/g to about 800 CE mg/g of polyphenols.

In one embodiment, the extract derived from sugar cane of the present disclosure comprises from about 10 CE g/L to about 70 CE g/L of polyphenols or from about 100 CE mg/g to about 700 CE mg/g of polyphenols.

In one embodiment, the extract derived from sugar cane of the present disclosure comprises from about 10 CE g/L to about 60 CE g/L of polyphenols or from about 100 CE mg/g to about 600 CE mg/g of polyphenols.

In one embodiment, the extract derived from sugar cane of the present disclosure comprises from about 10 CE g/L to about 50 CE g/L of polyphenols or from about 100 CE mg/g to about 500 CE mg/g of polyphenols.

In one embodiment, the extract derived from sugar cane of the present disclosure comprises from about 10 CE g/L to about 25 CE g/L of polyphenols or from about 100 CE mg/g to about 250 CE mg/g of polyphenols.

In one embodiment, the extract derived from sugar cane of the present disclosure comprises from about 15 CE g/L to about 50 CE g/L of polyphenols or from about 150 CE mg/g to about 500 CE mg/g of polyphenols.

In one embodiment, the extract derived from sugar cane of the present disclosure comprises from about 15 CE g/L to about 25 CE g/L of polyphenols or from about 150 CE mg/g to about 250 CE mg/g of polyphenols.

In one embodiment, the extract derived from sugar cane of the present disclosure comprises from about 15 CE g/L to about 40 CE g/L of polyphenols or from about 150 CE mg/g to about 400 CE mg/g of polyphenols.

In one embodiment, the extract derived from sugar cane of the present disclosure comprises from about 20 CE g/L to about 30 CE g/L of polyphenols or from about 200 CE mg/g to about 300 CE mg/g of polyphenols.

In one embodiment, the extract derived from sugar cane of the present disclosure comprises from about 20 CE g/L to about 27 g CE/L of polyphenols or from about 200 CE mg/g to about 270 CE mg/g of polyphenols.

In one embodiment, the extract derived from sugar cane of the present disclosure comprises from about 27 CE g/L to about 35 g CE/L of polyphenols or about 270 CE mg/g to about 350 CE mg/g of polyphenols.

In one embodiment, the extract derived from sugar cane of the present disclosure comprises from about 35 CE g/L to about 40 g CE/L of polyphenols or from about 350 CE mg/g to about 400 CE mg/g of polyphenols.

In one embodiment, the extract derived from sugar cane of the present disclosure comprises from about 40 CE g/L to about 50 g CE/L of polyphenols or from about 400 CE mg/g to about 500 CE mg/g of polyphenols.

In one embodiment, the extract derived from sugar cane of the present disclosure comprises from about 45 CE g/L to about 50 g CE/L of polyphenols or about 450 CE mg/g to about 500 CE mg/g of polyphenols.

The extract derived from sugar cane of the present disclosure may contain the flavonoid class of polyphenols. The extract derived from sugar cane may contain flavonoids in any amount. In one embodiment, the extract derived from sugar cane of the disclosure comprises at least about 1 CE g/L of flavonoids or at least about 10 CE mg/g of flavonoids.

In one embodiment, the extract derived from sugar cane of the present disclosure comprises from about 1 CE g/L to about 15 CE g/L of flavonoids or from about 10 CE mg/g to about 150 CE mg/g of flavonoids. In one embodiment, the extract derived from sugar cane of the present disclosure comprises from about 3 CE g/L to about 10 CE g/L of flavonoids or about 30 CE mg/g to about 100 CE mg/g of flavonoids. In one embodiment, the extract derived from sugar cane of the present disclosure comprises about 5 CE g/L to about 8 CE g/L of flavonoids or about 50 CE mg/g to about 80 CE mg/g of flavonoids. In one embodiment, the extract derived from sugar cane of the present disclosure comprises about 6 CE g/L to about 8 CE g/L of flavonoids or about 60 CE mg/g to about 80 CE mg/g of flavonoids. In one embodiment, the extract derived from sugar cane of the present disclosure comprises about 6.5 CE g/L to about 7.5 CE g/L of flavonoids or about 65 CE mg/g to about 75 CE mg/g of flavonoids.

The extract derived from sugar cane of the present disclosure may contain the proanthocyanidin class of polyphenols. The extract derived from sugar cane may contain proanthocyandins in any amount. In one embodiment, the extract derived from sugar cane of the present disclosure comprises at least about 1.5 CE g/L of proanthocyanidins or at least about 15 CE mg/g of proanthocyanidins. In one embodiment, the extract derived from sugar cane of the disclosure comprises at least about 1.8 CE g/L of proanthocyanidins or at least about 18 CE mg/g of proanthocyanidins. In one embodiment, the extract derived from sugar cane of the disclosure comprises about 1.5 CE g/L to about 2.5 CE g/L of proanthocyanidins or about 15 CE mg/g to about 25 CE mg/g of proanthocyanidins. In one embodiment, the extract derived from sugar cane of the disclosure comprises about 1.8 CE g/L to about 2.2 CE g/L of proanthocyanidins or about 18 CE mg/g to about 22 CE mg/g of proanthocyanidins.

The polyphenols of the extract derived from sugar cane of the present disclosure include, but are not limited to, one or more of syringic acid, chlorogenic acid, caffeic acid, vanillin, sinapic acid, p-coumaric acid, ferulic acid, gallic acid, vanillic acid, diosmin, diosmetin, apigenin, vitexin, orientin, homoorientin, swertisin, tricin, (+)-catechin, (-)-catechin gallate, (-) epicatechin, quercetin, kaempherol, myricetin, rutin, schaftoside, isoschaftoside, luteolin, scoparin and/or derivatives thereof. The polyphenols of the extract derived from sugar cane of the present disclosure may also include, but are not limited to, one or more of hydroxycinnamic acid, isoorientin, swertiajaponin, neocarlinoside, isovitexin, vicenin, and/or derivatives thereof.

The polyphenols of the extract derived from sugar cane also include conjugates, such as, for example, glycosides, glucosides, galactosides, galacturonides, ethers, esters, arabinosides, sulphates, phosphates, aldopentoses (xylose, arabinose) and aldohexoses.

In one embodiment, the extract derived from sugar cane of the present disclosure comprises syringic acid, chlorogenic acid, caffeic acid, vanillin, sinapic acid, diosmin, diosmetin, apigenin, vitexin, orientin, homoorientin, swertisin, and tricin and/or derivatives thereof.

In one embodiment, the extract derived from sugar cane of the present disclosure comprises syringic acid, chlorogenic acid and diosmin and/or derivatives thereof.

In one embodiment, the extract derived from sugar cane of the present disclosure comprises syringic acid. In one embodiment, the extract derived from sugar cane of the present disclosure comprises chlorogenic acid. In one embodiment, the extract derived from sugar cane of the present disclosure comprises diosmin. In one embodiment, the extract derived from sugar cane of the present disclosure comprises caffeic acid. In one embodiment, the extract derived from sugar cane of the present disclosure comprises vanillin. In one embodiment, the extract derived from sugar cane of the present disclosure comprises sinapic acid. In one embodiment, the extract derived from sugar cane of the present disclosure comprises vitexin. In one embodiment, the extract derived from sugar cane of the present disclosure comprises p-coumaric acid. In one embodiment, the extract derived from sugar cane of the present disclosure comprises ferulic acid. In one embodiment, the extract derived from sugar cane of the present disclosure comprises gallic acid. In one embodiment, the extract derived from sugar cane of the present disclosure comprises vanillic acid. In one embodiment, the extract derived from sugar cane of the present disclosure comprises diosmetin. In one embodiment, the extract derived from sugar cane of the present disclosure comprises apigenin. In one embodiment, the extract derived from sugar cane of the present disclosure comprises orientin. In one embodiment, the extract derived from sugar cane of the present disclosure comprises homoorientin. In one embodiment, the extract derived from sugar cane of the present disclosure comprises swertisin. In one embodiment, the extract derived from sugar cane of the present disclosure comprises tricin. In one embodiment, the extract derived from sugar cane of the present disclosure comprises (+)-catechin. In one embodiment, the extract derived from sugar cane of the present disclosure comprises (-)-catechin gallate. In one embodiment, the extract derived from sugar cane of the present disclosure comprises (-)-epicatechin. In one embodiment, the extract derived from sugar cane of the present disclosure comprises quercetin. In one embodiment, the extract derived from sugar cane of the present disclosure comprises kaempherol. In one embodiment, the extract derived from sugar cane of the present disclosure comprises myricetin. In one embodiment, the extract derived from sugar cane of the present disclosure comprises rutin. In one embodiment, the extract derived from sugar cane of the present disclosure comprises schaftoside. In one embodiment, the extract derived from sugar cane of the present disclosure comprises isoschaftoside. In one embodiment, the extract derived from sugar cane of the present disclosure comprises luteolin. In one embodiment, the extract derived from sugar cane of the present disclosure comprises scoparin. In one embodiment, the extract derived from sugar cane of the present disclosure comprises hydroxycinnamic acid. In one embodiment, the extract derived from sugar cane of the present disclosure comprises isoorientin. In one embodiment, the extract derived from sugar cane of the present disclosure comprises swertiajaponin. In one embodiment, the extract derived from sugar cane of the present disclosure comprises neocarlinoside. In one embodiment, the extract derived from sugar cane of the present disclosure comprises isovitexin. In one embodiment, the extract derived from sugar cane of the present disclosure comprises vicenin.

In one embodiment, syringic acid, chlorogenic acid and diosmin are the three most abundant polyphenols of the extract derived from sugar cane of the present disclosure.

In one embodiment, the extract derived from sugar cane of the disclosure comprises about 5 - 20 µg/g dry weight of syringic acid. In one embodiment, the extract derived from sugar cane of the disclosure comprises about 7 - 15 µg/g dry weight of syringic acid. In one embodiment, the extract derived from sugar cane of the disclosure comprises about 10 - 12 µg/g dry weight of syringic acid. In one embodiment, the extract derived from sugar cane of the disclosure, when present, comprises about 10.9 µg/g dry weight of syringic acid. The extract derived from sugar cane may be in a syrup form.

In one embodiment, the extract derived from sugar cane of the disclosure comprises about 50 - 200 µg/g dry weight of syringic acid. In one embodiment, the extract derived from sugar cane of the disclosure comprises about 90 - 130 µg/g dry weight of syringic acid. In one embodiment, the extract derived from sugar cane of the disclosure comprises about 100 - 120 µg/g dry weight of syringic acid. In one embodiment, the extract derived from sugar cane of the disclosure comprises about 107 µg/g dry weight of syringic acid. The extract derived from sugar cane may be in a powder form.

In one embodiment, the extract derived from sugar cane of the disclosure comprises about 1 - 15 µg/g dry weight of chlorogenic acid. In one embodiment, the extract derived from sugar cane of the disclosure comprises about 3 - 10 µg/g dry weight of chlorogenic acid. In one embodiment, the extract derived from sugar cane of the disclosure comprises about 5 - 8 µg/g dry weight of chlorogenic acid. In one embodiment, the extract derived from sugar cane of the disclosure comprises about 6.53 µg/g dry weight of chlorogenic acid. The extract derived from sugar cane may be in a syrup form.

In one embodiment, the extract derived from sugar cane of the disclosure comprises about 30 - 150 µg/g dry weight of chlorogenic acid. In one embodiment, the extract derived from sugar cane of the disclosure comprises about 60 - 90 µg/g dry weight of chlorogenic acid. In one embodiment, the extract derived from sugar cane of the disclosure comprises about 70 - 80 µg/g dry weight of chlorogenic acid. In one embodiment, the extract derived from sugar cane of the disclosure comprises about 74 µg/g dry weight of chlorogenic acid. The extract derived from sugar cane may be in a powder form.

In one embodiment, the extract derived from sugar cane of the disclosure comprises about 10 - 30 µg/g dry weight of diosmin. In one embodiment, the extract derived from sugar cane of the disclosure comprises about 15 - 25 µg/g dry weight of diosmin. In one embodiment, the extract derived from sugar cane of the disclosure comprises about 18 - 21 µg/g dry weight of diosmin. In one embodiment, the extract derived from sugar cane of the disclosure comprises about 19 - 45 µg/g dry weight of diosmin. The extract derived from sugar cane may be in a syrup form.

In one embodiment, the extract derived from sugar cane of the disclosure comprises about 100 - 300 µg/g dry weight of diosmin. In one embodiment, the extract derived from sugar cane of the disclosure comprises about 190 - 260 µg/g dry weight of diosmin. In one embodiment, the extract derived from sugar cane of the disclosure comprises about 210 - 240 µg/g dry weight of diosmin. In one embodiment, the extract derived from sugar cane of the disclosure comprises about 227 µg/g dry weight of diosmin. The extract derived from sugar cane may be in a powder form.

In one embodiment, the extract derived from sugar cane of the present disclosure comprises about 7 - 15 µg/g dry weight of syringic acid, and/or about 4 - 9 µg/g dry weight of chlorogenic acid, and/or about 0.1 - 0.5 µg/g dry weight of caffeic acid, about 0.05 - 0.3 µg/g dry weight of vanillin, and/or about 0.1 - 0.3 µg/g dry weight of sinapic acid, and/or about 15 - 25 µg/g dry weight of diosmin, and/or about 0.1 - 0.4 µg/g dry weight of orientin, and/or about 0.4-0.9 µg/g dry weight of swertisin, and/or about 0.05 - 0.3 µg/g dry weight of disomentin. The extract derived from sugar cane may be in a syrup form.

In one embodiment, the extract derived from sugar cane of the present disclosure comprises about 10 - 12 µg/g dry weight of syringic acid, and/or about 5 - 8 µg/g dry weight of chlorogenic acid, and/or about 0.2 - 0.4 µg/g dry weight of caffeic acid, and/or about 0.1 - 0.2 µg/g dry weight of vanillin, and/or about 0.1 - 0.25 µg/g dry weight of sinapic acid, and/or about 18 - 21 µg/g dry weight of diosmin, and/or about 0.2 - 0.3 µg/g dry weight of orientin, and/or about 0.5-0.8 µg/g dry weight of swertisin, and/or about 0.1 - 0.2 µg/g dry weight of disomentin. The extract derived from sugar cane may be in a syrup form.

In one embodiment, the extract derived from sugar cane of the present disclosure comprises about 10.9 µg/g dry weight of syringic acid, and/or about 6.53 µg/g dry weight of chlorogenic acid, and/or about 0.29 µg/g dry weight of caffeic acid, and/or about 0.153 µg/g dry weight of vanillin, and/or about 0.18 µg/g dry weight of sinapic acid, and/or about 19.45 µg/g dry weight of diosmin, and/or about 0.245 µg/g dry weight of orientin, and/or about 0.69 µg/g dry weight of swertisin, and/or about 0.15 µg/g dry weight of disomentin. The extract derived from sugar cane may be in a syrup form.

In one embodiment, the extract derived from sugar cane of the present disclosure comprises about 90 - 130 µg/g dry weight of syringic acid, and/or about 60 - 90 µg/g dry weight of chlorogenic acid, and/or about 4 - 10 µg/g dry weight of caffeic acid, and/or about 1 - 4 µg/g dry weight of vanillin, about 1 - 3 µg/g dry weight of sinapic acid, and/or about 190 - 260 µg/g dry weight of diosmin, and/or about 3 - 7 µg/g dry weight of orientin, and/or 3 - 8 µg/g dry weight of swertisin, and/or about 0.05 - 0.3 µg/g dry weight of disomentin. The extract derived from sugar cane may be in a powder form.

In one embodiment, the extract derived from sugar cane of the present disclosure comprises about 100 - 120 µg/g dry weight of syringic acid, and/or about 70 - 80 µg/g dry weight of chlorogenic acid, and/or about 6 - 8 µg/g dry weight of caffeic acid, about 2 - 3 µg/g dry weight of vanillin, and/or about 1.5 - 2.5 µg/g dry weight of sinapic acid, and/or about 210 - 240 µg/g dry weight of diosmin, about 4 - 5 µg/g dry weight of orientin, 4-6 µg/g dry weight of swertisin, and/or about 0.1 - 0.2 µg/g dry weight of disomentin. The extract derived from sugar cane may be in a powder form.

In one embodiment, the extract derived from sugar cane of the present disclosure comprises about 107 µg/g dry weight of syringic acid, and/or about 74 µg/g dry weight of chlorogenic acid, and/or about 7.5 µg/g dry weight of caffeic acid, and/or about 2 µg/g dry weight of vanillin, and/or about 1.7 µg/g dry weight of sinapic acid, and/or about 227 µg/g dry weight of diosmin, and/or about 4.5 µg/g dry weight of orientin, 5.2 µg/g dry weight of swertisin, and/or about 0.16 µg/g dry weight of disomentin. The extract derived from sugar cane may be in a powder form.

The extract derived from sugar cane of the present disclosure may contain a range of organic acids that are found naturally in sugar cane. These organic acids may include, but are not limited to, aconitic (*cis-* and *trans*-), oxalic, citric, lactic, tartaric, glycolic, succinic, citric, malic, fumaric and shikimic acids. In one embodiment, the extract derived from sugar cane contains higher levels of citric and malic acids than other organic acids. In another embodiment, the extract derived from sugar cane contains low to trace amounts of oxalic, citric, tartaric, glycolic, succinic and citric acids. In another embodiment, the two most abundant organic acids in the extract derived from sugar cane are *trans-* and cis-aconitic acids.

The extract derived from sugar cane of the present disclosure may contain *trans-* and/or cis-aconitic acids. In one embodiment, the extract derived from sugar cane of the present disclosure comprises trans-aconitic in amount of about 10,000 - 40,000 mg per kg and/or cis-aconitic in amount of about 3,000 - 7,000 mg/kg. In one embodiment, the extract derived from sugar cane of the present disclosure contains *trans*-aconitic in an amount of about 17,000 - 30,000 mg per kg and/or *cis*-aconitic in amount of about 4,000 - 6,500 mg/kg. In one embodiment, the extract derived from sugar cane of the present disclosure may contain *trans*-aconitic in amount of about 20,000-25,000 mg per kg and/or *cis*-aconitic in amount of about 5,000 - 5,500 mg/kg.

The extract derived from sugar cane of the present disclosure may contain amino acids. In one embodiment, the total amino acids levels of the extract derived from sugar cane of the present disclosure is about 50,000 - 80,000 µg per gram, or about 60,000 - 70,000 µg per gram, or about 65,000 µg per gram. In one embodiment, about 10 - 40% of these total amino acids are essential amino acids. In one embodiment, about 15 - 30% of these total amino acids are essential amino acids. In one embodiment, about 20 - 25% of these total amino acids are essential amino acids.

The extract derived from sugar cane of the present disclosure may contain free amino acids. In one embodiment, the extract derived from sugar cane of the present disclosure comprises about 10,000 - 50,000 µg of free amino acids per gram. In one embodiment, the extract derived from sugar cane of the present disclosure may contain about 20,000 - 35,000 µg of free amino acids per gram. The extract derived from sugar cane of the present disclosure may contain about 25,000 - 30,000 µg of free amino acids per gram.

As defined above, the term "free amino acids" as used herein refers to amino acids which are singular molecules and structurally not attached to peptide bonds which are attached to other amino acids.

The extract derived from sugar cane of the present disclosure may contain leucine, a branched chain essential amino acid. In one embodiment, the concentration of leucine in the extract derived from sugar cane, is about 1 - 5 mM, or about 1.5 - 4 mM, or about 2 - 3 mM. In one embodiment, the amount of leucine in the extract derived from sugar cane is about 1,000 - 20,000 µg per gram, or about 1,000 - 10,000 µg per gram, or about 1,000 - 5,000 µg per gram, or about 1,000 - 2,000 µg per gram, or about 5,000 - 10,000 µg per gram, or about 10,000 - 20,000 µg per gram.

The extract derived from sugar cane of the present disclosure may contain minerals. In one embodiment, the extract derived from sugar cane contains minerals that are found naturally in sugar cane. In one embodiment, the extract derived from sugar cane contains one or more minerals including, but not limited to, potassium, sodium, calcium, magnesium, iron, zinc, selenium and chromium.

In one embodiment, the extract derived from sugar cane contains minerals bound to the polyphenols. In one embodiment, the extract derived from sugar cane contains divalent ions bound to the polyphenols. In one embodiment, the extract derived from sugar cane contains calcium, magnesium and/or iron bound to the polyphenols. In one embodiment, the extract derived from sugar cane contains iron bound to the polyphenols.

In one embodiment, the extract derived from sugar cane of the present disclosure comprises about 20,000 - 32,000 mg of potassium per kilogram, and/or about 300 - 600 mg of sodium per kilogram, and/or about 800 - 1,300 mg of calcium per kilogram, and/or about 3,000 - 6,000 mg of magnesium per kilogram, and/or about 40 - 90 mg of iron per kilogram, and/or about 3 - 10 mg of zinc per kilogram, and/or about 500 - 900 µg of selenium per kilogram and/or about 1,000 - 1,600 µg of chromium per kilogram. The extract derived from sugar cane may be in a syrup form.

In one embodiment, the extract derived from sugar cane of the present disclosure comprises about 25,000 - 27,000 mg of potassium per kilogram, and/or about 400 - 500 mg of sodium per kilogram, and/or about 1,000 - 1,200 mg of calcium per kilogram, and/or about 4,000 - 5,500 mg of magnesium per kilogram, and/or about 55 - 75 mg of iron per kilogram, and/or about 5.5 - 7.5 mg of zinc per kilogram, and/or about 700 - 850 µg of selenium per kilogram, and/or about 1,200 - 1,400 µg of chromium per kilogram. The extract derived from sugar cane may be in a syrup form.

In one embodiment, the extract derived from sugar cane of the present disclosure comprises about 26,000 mg of potassium per kilogram, and/or about 450 mg of sodium per kilogram, and/or about 1,090 mg of calcium per kilogram, and/or about 4,700 mg of magnesium per kilogram, and/or about 65 mg of iron per kilogram, about 6.6 mg of zinc per kilogram, and/or about 786 µg of selenium per kilogram and/or about 1,300 µg of chromium per kilogram. The extract derived from sugar cane may be in a syrup form.

In one embodiment, the extract derived from sugar cane of the present disclosure comprises about 50 - 350 mg of potassium per kilogram, and/or about 5 - 70 mg of sodium per kilogram, and/or about 7,000 - 10,000 mg of calcium per kilogram, and/or about 1,000 - 3,000 mg of magnesium per kilogram, and/or about 500 - 1,300 mg of iron per kilogram. The extract derived from sugar cane may be in a powder form.

In one embodiment, the extract derived from sugar cane of the present disclosure comprises about 100 - 250 mg of potassium per kilogram, and/or about 10 - 50 mg of sodium per kilogram, and/or about 8,000 - 9,000 mg of calcium per kilogram, and/or about 1,500 - 2,500 mg of magnesium per kilogram, and/or about 800 - 1,000 mg of iron per kilogram. The extract derived from sugar cane may be in a powder form.

In one embodiment, the extract derived from sugar cane of the present disclosure comprises about 190 mg of potassium per kilogram, and/or about 30 mg of sodium per kilogram, and/or about 8,800 mg of calcium per kilogram, and/or about 2,000 mg of magnesium per kilogram, and/or about 890 mg of iron per kilogram. The extract derived from sugar cane may be in a powder form.

The extract derived from sugar cane of the present disclosure may contain monosaccharides, disaccharides, oligosaccharides and/or polysaccharides. Examples of these include, but are not limited to, sucrose, glucose, galactose, xylose, ribose, mannose, rhamnose, fructose, maltose, lactose, maltotriose, xylopyranose, raffinose, 1-kestose, theanderose, 6-kestose, panose, neo-kestose, nystose, glucans and xylans.

In one embodiment, the compositions and methods comprises from about 0.001 wt% to about 10 wt% of the extract.

In one embodiment, the composition and methods comprises from about 5 wt% to about 10 wt% of the extract.

In one embodiment, the composition and methods comprises from about 0.05 wt% to about 10 wt% of the extract.

In one embodiment, the composition and methods comprises from about 0.05 wt% to about 5 wt% of the extract.

In one embodiment, the agricultural greenhouse gas emissions are caused by Archael methanogens.

In one embodiment, the composition may be included in a ruminant animal feed.

The preparation of the extracts of sugar cane of this disclosure has been extensively described in WO 2019/028506. In particular page 17 to page 40 line 22 of WO 2019/028506, describe the preparation of these extracts.

Similarly in WO 2019/028506 page 50 line 16 to page 51 line 24, compositions of the extracts of sugar cane are described. Further, in WO 2019/028506 page 64 to page 88 line 9, the characteristics of the extracts of sugar cane are described.

More generally, the disclosure of WO 2019/028506 is also relevant.

A detailed analysis of a sample a sugar cane extract of this disclosure has revealed the following components:
Chlorogenic acid 2423 ng/mL
Vanillic acid 16088 ng/mL
Caffeic acid 482 ng/mL
Trans caffeic acid 273 ng/mL
p Coumaric acid 14445 ng/mL
In addition, luteolin, trans resveratrol, diosmetin, swertisin and rutin were found to be present along with the flavonoids, apigenin-6-C-arabinosyl-8-C glucoside, apigenin-6-C-glucosyl-8-C arabinoside, apigenin-6, 8-C-diglucoside, apigenin-6"-O-glucosyl-8-C glucoside, schaftoside-2"-O-glucoside, apigenin-6-C-rhamnosyl-8-C glucoside, apigenin-6, 8-C-diarabinoside, methoxyluteolin-8-C-glucoside, methoxyluteolin-6"-O-rutinosyl-8-C-glucoside, methoxyluteolin-6"-O-glucosyl-8-C-glucoside, tricin 7-O-neohesperidoside, tricin 7-O-rhamnosyl-glucuronide and tricin 7-O-glycoside.

Spray drying of extracts of this disclosure may be prepared by known methods. Such methods may or may not include a carrier such as a maltodextrin.

In one embodiment the extracts may be spray dried at 100% concentration with the spray drier inlet temperature at 140-150°C and an outlet temperature at 90-95°C.

In one embodiment the extracts may be spray dried at 80% concentration using a maltodextrin carrier with the spray drier inlet temperature at 150-160°C and an outlet temperature at 90-95°.

Compositions suitable for use in non-human animals have been extensively described in WO 2019/028506, for example at [238] to [251] and [253] to [254].

In one embodiment, in compositions suitable for use in ruminants, an extract of this disclosure may be incorporated in feeds at a concentration of 0.001%-10% w/w preferably 0.01%-5%, most preferably 0.1%-2% or % w/v for a liquid feed in order to achieve methane reductions of 3 to 80%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve methane reductions of 5 to 70%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve methane reductions of 10 to 60%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve methane reductions of 5 to 50%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve methane reductions of 5 to 40%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve methane reductions of 10 to 30%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve methane reductions of 5 to 10%.

In one embodiment, in compositions suitable for use in ruminants, an extract of this disclosure may be incorporated in feeds at a concentration of 0.001%-10% w/w preferably 0.01%-5%, most preferably 0.1%-2% or w/v for a liquid feed in order to achieve in order to achieve enhanced milk yields to 1 to 15% or 2 to 10% or 3 to 7%.

An example 1 of the preparation of the extracts of this disclosure will now be described. This extract is designated Polygain.
- Vinasse is stored in tanks with recirculation prior to use.
- Vinasse feedstock is passed through a 10 micron mesh to remove any large particles.
- The Feedstock is then subjected to high shear mixing.
- Following mixing, it is heated to 55°C.
- The heated material is filtered through a 0.1mircon membrane filtration system.
- Following filtration, it is evaporated to 60 brix.
- If it is to be stored as a liquid, it is heated to 80°C and packed.
- If spray dried material is required, the 60 brix product is blended with maltodextrin and mixed well.
- The so-formed slurry is spray dried with inlet temperature range of 150-160°C and outlet temperature range of 90-95°.

The polyphenol content of the Polygain was determined as follows:

| Polyphenol | Molecular formula |
|---|---|
| Chlorogenic acid | C₁₆H₁₈O₉ |
| Trans-caffeic & Caffeic acid | C₉H₈O₄ |
| Syringic acid | C₉H₁₀O₅ |
| Trans-p-coumaric | C₉H₈O₃ |
| Trans sinapic acid | C₁₁H₁₂O₅ |
| Vitexin | C₂₁H₂₀O₁₀ |
| Swertisin | C₂₂H₂₂O₁₀ |
| Homoorientin & Orientin | C₂₁H₂₀O₁₁ |
| Quercetin | C₁₅H₁₀O₇ |
| Apigenin | C₁₅H₁₀O₅ |
| Tricin | C₂₃H₂₄O₁₂ |

An example 2 of the preparation of the extracts of this disclosure will now be described. This extract is designated Virofonol.
1. Mix 300kg of sugar cane extract with 900L of hot water (80-85°C).
2. Mix well and check brix is 20 then add to settling tank with FPX-66 resin.
3. Mix for 20 minutes then allow the resin to settle for 15-20 minutes.
4. Pass through a 200 micron pre-filter into a tank.
5. Drain and dispose of the remaining liquid.
6. Rinse the resin with approximately 1000L of cold water. Mix well and drain.
7. Add ethanol 71-76% to the settling tank with resin.
8. Mix for 20 minutes then allow the resin to settle for 15-20 minutes.
9. Pass through 200 micron pre-filter into a tank and collect the liquid.
10. Repeat steps 7-9
11. Once all the liquid is collected, add 500L of warm water (35-40°) to the resin.
12. Mix for 20 minutes then allow the product to settle for 15-20 minutes.
13. Drain and collect the liquid.
14. Add all the liquid collected into an evaporator and concentrate to 45 Brix.
15. Spray dry 45 Brix liquid with inlet temperature range of 140-150°C and the outlet temperature range of 90-95°C sieve and pack.

| **Concentration (mg/ml)** | **% Reduction** |
|---|---|
| **Polygain: 2** | **36.91** |

In a further example 13, a composition of this disclosure (Polygain) was evaluated for its ability to reduce methane emissions by dry cows.

Dry cows were fed 3 kg concentrate mix (control) or concentrate mixed with 100g Polygain. Measurements of methane concentration were determined at 11 am and again at 1 pm.

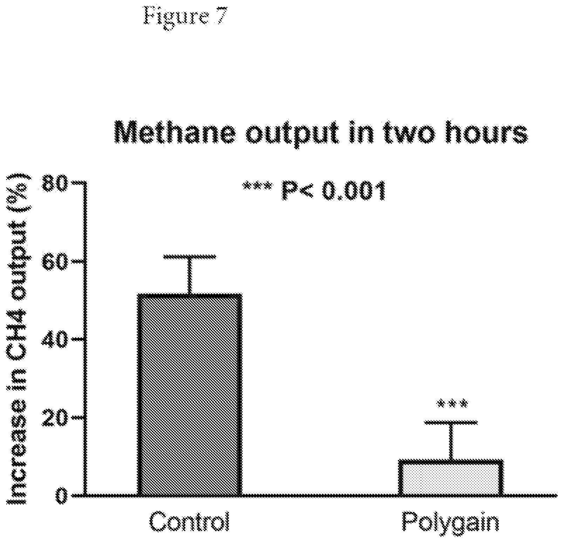

The results in figure 7 clearly show that Polygain reduces methane emission significantly during the first two hours when compared to the control group. These results demonstrate the dysbiosis effect of the compositions of this disclosure.

In a further example 14 a composition of this disclosure (Polygain) was evaluated for its ability to reduce methane generation *in vitro.* In this example, a sample of rumen fluid was obtained and used to ferment samples of a typical feedlot diet or Rhodes grass. In the sample under evaluation, a concentration of 15% of Polygain was included in each of the fermentations.

The results as set out below show that for the feedlot diet, a 54% reduction in methane generation was achieved after 24 hours per gram of digested dry matter. For the Rhode grass diet, the reduction was 16.5% after 24 hours per gram of digested dry matter.

| | **Feedlot** | | **Rhode grass** | |
|---|---|---|---|---|
| **Parameter** | 0% | 15% | 0% | 15% |
| Cumulative CH₄ 24h, mL | 4.44 | 3.73 | 4.14 | 5.07 |
| Cumulative CH₄ 24h, mL/g digested DM | 10.90 | 5.06 | 7.88 | 6.58 |

| Dependent Variable | Treatment | Mean |
|---|---|---|
| Inter-quartile ratio difference over trial | Control | 0.016 |
| | Polygain | -0.006 |

In a further example 19, a composition of this disclosure (Polygain) was evaluated for its ability to reduce methane emissions by dry cows. Polygain was included in the feed for each of the test groups of cows at 0.5% or 1% w/w on a dry matter basis.

At the commencement of the trial, methane concentrations were captured at periodic times over about a 42 hour period. Methane concentrations were again captured after 32 days of feeding at periodic times over about a 42 hour period.

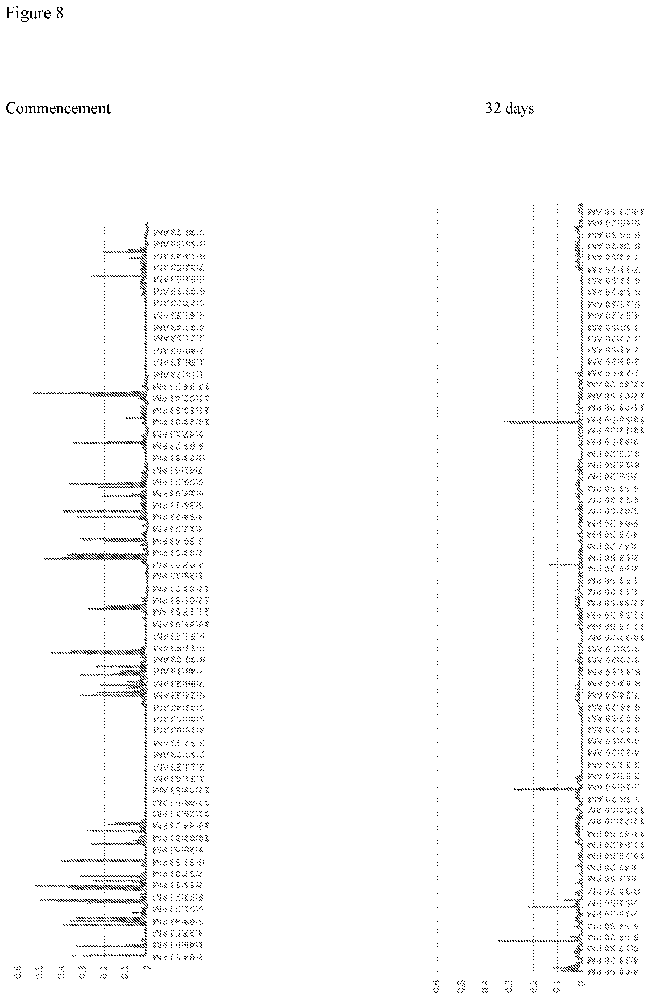

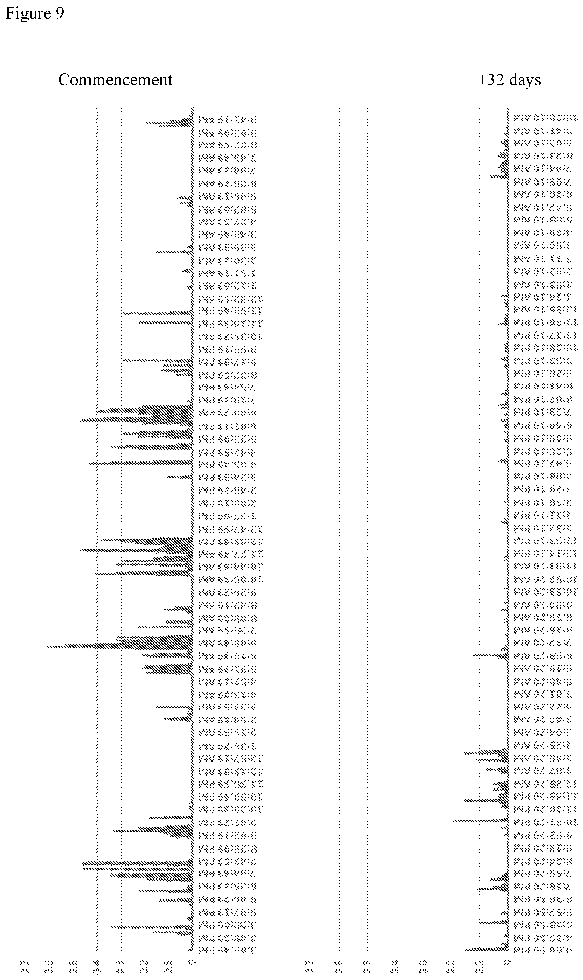

The results of the trial at 0.5% Polygain are shown in figure 8 and the results for 1% Polygain are shown in figure 9. Referring to figure 8, qualitatively it can be seen that the methane concentration in the 0.5% test group recorded four values only above 0.2. By comparison at commencement, numerous values in excess of 0.2 were recorded.

Referring to figure 9, qualitatively it can be seen that the methane concentration in the 1% test group did not exceed 0.2. By comparison at commencement, numerous values in excess of 0.2 were recorded.

Thus this example 19 shows clearly the dysbiosis effect compositions of this disclosure.

In a further example 20, the faeces of a cow that had been fed on a control diet and a second cow that had been fed on the same diet with the addition of 100g Polygain were evaluated for methanogenic archea populations.

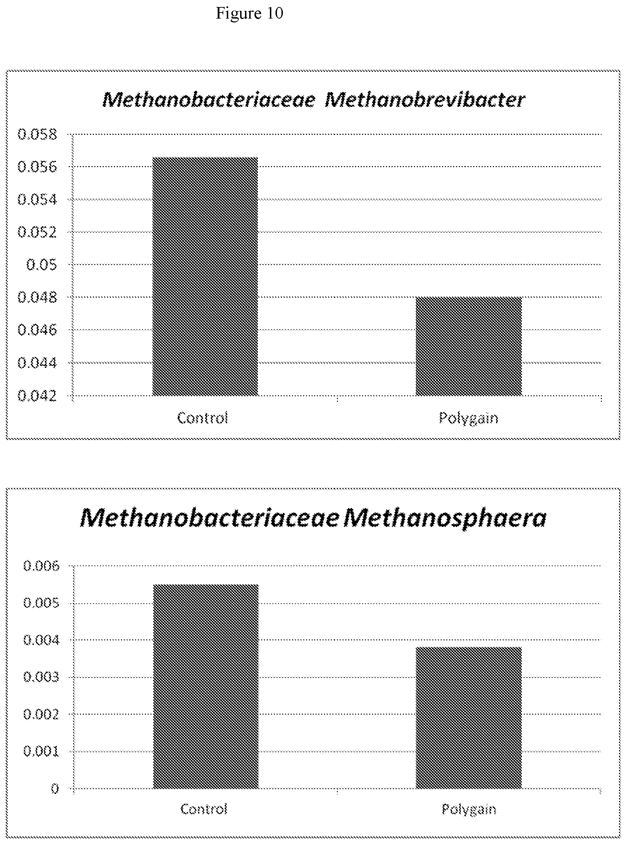

The results of example 20 are depicted in figure 10. These show that for Methanobacteriaceae Methanobrevibacter, the reduction in population of this microorganism was about 15% whilst for Methanobacteriaceae Methanosphaera, the reduction was about 30%. Such results reinforce the findings in other examples disclosed here that the methane reducing effect of Polygain is due to changes induced in the microbiome of ruminants. Specifically, the populations of methane generating microorganisms.

## Claims

1. Use of a composition in reducing methane production by a ruminant animal by changing the composition of microbial communities associated with the rumen of the ruminant animal comprising administering to the ruminant animal, an effective amount of a composition comprising from 0.001 wt% to 10 wt % of an extract derived from sugar cane to reduce methane production in the ruminant animal by 3-80%, the extract comprising from 10 catechin equivalent (CE) g/L to 100 CE g/L of polyphenols or from 100 CE mg/g to 1000 CE mg/g of polyphenols.

2. The use according to claim 1, wherein the extract comprises:
from 10 catechin equivalent (CE) g/L to 80 CE g/L of polyphenols or from 100 CE mg/g to 800 CE mg/g of polyphenols; or
from 10 CE g/L to 50 CE g/L of polyphenols or from 100 CE mg/g to 500 CE mg/g of polyphenols.

3. The use according to claim 1 or claim 2 wherein the extract comprises:
from 1 CE g/L to 15 CE g/L of flavonoids; or from 10 CE mg/g to 150 CE mg/g of flavonoids.

4. The use according to any one of claims 1 to 3, wherein the composition is a feed for ruminants comprising:
from 0.01 wt% to 5 wt % or 0.01 w/v % to 5 w/v % of the extract; or
from 0.1 wt% to 2 wt % or 0.1 w/v % to 2 w/v % othe extract.

5. The use according to claim 4, wherein methane production by an animal is reduced by 10-60 %; or reduced by 10-30 %; or reduced by 5-70 %; or reduced by 5-50 %; or reduced by 5-40 %; or reduced by 5-10 %.

## Patentansprüche

1. Verwendung einer Zusammensetzung zur Reduktion der Methanproduktion durch ein wiederkäuendes Tier durch Verändern der Zusammensetzung von mikrobiellen Gemeinschaften in Verbindung mit dem Pansen des wiederkäuenden Tiers, welche die Verabreichung einer wirksamen Menge einer 0,001 Gew.-% bis 10 Gew.-% eines aus Zuckerrohr gewonnenen Extrakts umfassenden Zusammensetzung an den Wiederkäuer umfasst, um die Methanproduktion im Wiederkäuer um 3 bis 80 % zu reduzieren, wobei der Extrakt 10 Catechin-Äquivalente (CE) g/l bis 100 CE g/l Polyphenole oder 100 CE mg/g bis 1000 CE mg/g Polyphenole umfasst.

2. Verwendung nach Anspruch 1, wobei der Extrakt Folgendes umfasst:
10 Catechin-Äquivalente (CE) g/l bis 80 CE g/l Polyphenole oder 100 CE mg/g bis 800 CE mg/g Polyphenole; oder
10 CE g/l bis 50 CE g/l Polyphenole oder 100 CE mg/g bis 500 CE mg/g Polyphenole.

3. Verwendung nach Anspruch 1 oder Anspruch 2, wobei der Extrakt Folgendes umfasst:
1 CE g/l bis 15 CE g/l Flavonoide oder 10 CE mg/g bis 150 CE mg/g Flavonoide.

4. Verwendung nach einem der Ansprüche 1 bis 3, wobei die Zusammensetzung ein Futtermittel für Wiederkäuer ist, das Folgendes umfasst:
0,01 Gew.-% bis 5 Gew.-% oder 0,01 % (Gew./Vol.) bis 5 % (Gew./Vol.) des Extrakts; oder
0,1 Gew.-% bis 2 Gew.-% oder 0,1 % (Gew./Vol.) bis 2 % (Gew./Vol.) des Extrakts.

5. Verwendung nach Anspruch 4, wobei die Methanproduktion durch ein Tier um 10 bis 60 % reduziert wird; oder um 10 bis 30 % reduziert wird; oder um 5 bis 70 % reduziert wird; oder um 5 bis 50 % reduziert wird; oder um 5 bis 40 % reduziert wird; oder um 5 bis 10 % reduziert wird.

## Revendications

1. Utilisation d'une composition pour réduire la production de méthane par un animal ruminant en modifiant la composition de communautés microbiennes associées au rumen de l'animal ruminant, comprenant l'administration à l'animal ruminant d'une quantité efficace d'une composition comprenant de 0,001 % en poids à 10 % en poids d'un extrait dérivé de canne à sucre pour réduire la production de méthane chez l'animal ruminant de 3 à 80 %, l'extrait comprenant de 10 équivalents de catéchine (EC) g/L à 100 EC g/L de polyphénols ou de 100 EC mg/g à 1 000 EC mg/g de polyphénols.

2. Utilisation selon la revendication 1, dans laquelle l'extrait comprend :
de 10 équivalents de catéchine (EC) g/L à 80 EC g/L de polyphénols ou de 100 EC mg/g à 800 EC mg/g de polyphénols ; ou
de 10 EC g/L à 50 EC g/L de polyphénols ou de 100 EC mg/g à 500 EC mg/g de polyphénols.

3. Utilisation selon la revendication 1 ou la revendication 2 dans laquelle l'extrait comprend :
de 1 EC g/L à 15 EC g/L de flavonoïdes ; ou de 10 EC mg/g à 150 EC mg/g de flavonoïdes.

4. Utilisation selon l'une quelconque des revendications 1 à 3, dans laquelle la composition est un aliment pour ruminants comprenant :
de 0,01 % en poids à 5 % en poids ou de 0,01 % en poids/volume à 5 % en poids/volume de l'extrait ; ou
de 0,1 % en poids à 2 % en poids ou de 0,1 % en poids/volume à 2 % en poids/volume de l'extrait.

5. Utilisation selon la revendication 4, dans laquelle la production de méthane par un animal est réduite de 10 à 60 % ; ou réduite de 10 à 30 % ; ou réduite de 5 à 70 % ; ou réduite de 5 à 50 % ; ou réduite de 5 à 40 % ; ou réduite de 5 à 10 %.
